(19) Europäisches Patentamt
European Patent Office
Office européen des brevets

(11) Publication number: **0 303 995 A1**

# (12) EUROPEAN PATENT APPLICATION

(21) Application number: 88113171.8

(22) Date of filing: 12.08.88

(51) Int. Cl.4: A61K 7/42

(30) Priority: 17.08.87 JP 203147/87

(43) Date of publication of application:
22.02.89 Bulletin 89/08

(84) Designated Contracting States:
DE ES FR GB IT NL

(71) Applicant: KAO CORPORATION
1-14-10, Nihonbashikayaba-cho
Chuo-ku Tokyo103(JP)

(72) Inventor: Itoh, Hiroshi
2-1-13, Kajino-cho
Koganei-shi Tokyo(JP)
Inventor: Kashimoto, Akio
c/o Kao-Ryo, 3-20-1, Innai
Funabashi-shi Chiba-ken(JP)
Inventor: Fujii, Shigeo
1450, Nishihama
Wakayama-shi Wakayama-ken(JP)

(74) Representative: Wächtershäuser, Günter, Dr.
Tal 29
D-8000 München 2(DE)

## (54) Long wavelength UV ray absorber, process for preparing the same, and cosmetic containing the same.

(57) A long wavelength UV ray absorber and a cosmetic comprising the same is disclosed. The long wavelength UV ray absorber comprises (a) a dibenzoylmethane derivative of the type in which the benzene ring of the dibenzoylmethane has a carboxylic acid group, a carboxylate group, or a carboxylic acid ester group, and (b) a polyvalent metal oxide. The long wavelength UV ray absorber can effectively shut out UV-A which is harmful to the skin, thus protecting it from the UV-A. It is stable against light and heat over a long period of time. Since it is sparingly soluble into water, organic solvents, oily substances, or sabum, it is hardly absorbed through the skin and hardly removed from the skin by water or sweat.

EP 0 303 995 A1

Xerox Copy Centre

# LONG WAVELENGTH UV RAY ABSORBER, PROCESS FOR PREPARING THE SAME, AND COSMETIC CONTAINING THE SAME

## BACKGROUND OF THE INVENTION

### Field of the Invention:

This invention relates to a long wavelength UV ray absorber and, more particularly, to a long wavelength UV ray absorber comprising (a) a dibenzoylmethane derivative of the type in which the benzene ring of the dibenzoylmethane has a carboxylic acid group, a carboxylate group, or a carboxylic acid ester group, and (b) a polyvalent metal oxide. The invention also relates to a cosmetic containing such a long wavelength UV ray absorber.

### Description of the Background:

Ultraviolet rays are known to bring about a variety of changes to the skin. In dermatology, UV rays are classified into long wavelength UV rays of 400 - 320 nm, medium wavelength UV rays of 320 - 290 nm, and short wavelength UV rays of less than 290 nm. These are called UV-A, UV-B, and UV-C, respectively.

Most of the UV ray sources to which a human being is exposed are usually the sunlight. The UV rays which arrive at the ground are UV-A and UV-B, and UV-C rarely reaches the ground because it is absorbed in the ozone layer. Among the UV rays reaching the ground, the UV-B, when irradiated onto the skin in an amount exceeding a certain level, can cause red spots or blisters to be formed on it. In addition, the UV-B can promote melanosis on the skin, resulting in the pigmentation or the like changes of the skin. On the other hand, UV-A has conventionally been considered to cause no significant changes to the skin. Recent development in the electron microscope and histochemical techniques, however, has revealed that UV-A also produces some changes to the skin. In particular, unlike UV-B, UV-A energy reaches the corium and brings about slow-chronic changes in the blood vessel walls and the elastic fibers in connective tissues. These changes is considered to accelerate the aging of the skin. Furthermore, the UV-A is known to melanize the skin (instant melanism) immediately after irradiation and to promote the UV-B's action on the skin. Thus, UV-A is considered to be a cause of spots or freckles formation, or of the exacerbation of the skin. These factors indicate that protecting the skin not only from UV-B but also from UV-A is important to prevent the skin from being aged, to avert formation of spots or freckles, and to preclude the skin from being exacerbated.

Studies on the action of UV-A to the skin, however, have just started and few substance which is capable of effectively absorbing the UV-A is known, excepting for dibenzoylmethane derivatives and cinnamic acid derivatives (West Germany Patent Laid-open No. 2728241, West Germany Patent Laid-open No. 2728243, Japanese Patent Laid-open No. 61641/1976, Japanese Patent Laid-open No. 46056/1977, Japanese Patent Laid-open No. 197209/1982, and Japanese Patent Laid-open No. 59840/1982).

The inventors have previously made studies to develop a long wavelength UV ray absorber which satisfies the following requirements (1) through (8):

(1) having a maximum absorption wavelength in the vicinity of 350 nm.

(2) having a sufficiently high molar extinction coefficient ($\epsilon$) at the above maximum absorption wavelength.

(3) having a small absorbance in the visible light range, i.e., $\epsilon \fallingdotseq 0$ at above 400 nm (since a colored substance is not desirable for use as a cosmetic).

(4) having good stablity against heat and light.

(5) being free of toxicity, irritativeness, and any other harmful actions to the skin.

(6) having excellent compatibility with the cosmetic base material.

(7) upon application to the skin, being sparingly absorbed through the skin and removed only with difficulty by perspiration, etc., thus ensuring long-lasting effects.

(8) being inexpensive.

As a result, the inventors have found that a dibenzoylmethane derivative represented by the following formula (I) satisfied all of the above requirements:

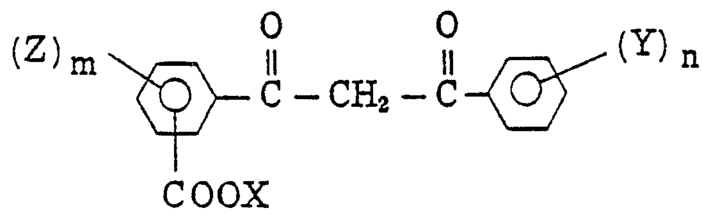
(I)

in which X represents a hydrogen atom, a monovalent metal cation, an organic cation, a linear or branched aliphatic hydrocarbon group of a $C_{1-24}$ carbon atom content, a polyoxyalkylene (the alkylene group having 2 or 3 carbon atoms) oxide group of a $C_{1-24}$ carbon atom content, Y and Z may be the same or different and represent a hydroxyl group, a linear or branched aliphatic hydrocarbon group of a $C_{1-24}$ carbon atom content, an alkoxy group of a $C_{1-24}$ carbon atom content, or a polyoxyalkylene (the alkylene group having 2 or 3 carbon atoms) oxide group, and m and n are independently an integer of 0 - 3. An application for patent was filed based on this finding (Japanese Patent Laid-open No. 190708/1985).

The inventors also found that the combination of this dibenzoylmethane derivative and a hydrated oxide of polyvalent metal provided a UV ray absorber which is stable both in water phase and in oil phase. A patent application was filed based on this discovery (Japanese Patent Laid-open No. 213280/1986). The inventors have further found that polyvalent metal salts of this dibenzoylmethane derivative (I) could be advantageously formulated into cosmetics or the like, because they were hardly absorbed through the skin because of their insolubility into water, organic solvents, oily substances, sebum, or the like, and because of their capability to resist being removed by water, sweat, or the like, and filed a patent application based on this finding (Japanese Patent Laid-open No. 54506/1986).

Increasing interest in recent years in protecting the skin from UV rays, however, brought forward the demand for formulating a UV ray absorber into make-up cosmetics, especially into foundations.

A UV ray absorber can be formulated into make-up cosmetic either by compounding the former into the oil phase or water phase, or by adding it in a powdery form. The compound of formula (I), however, either in itself or in combination with a hydrated polyvalent metal oxide, is not sufficiently soluble into the water or oil phase, and thus cannot effectively be incorporated into either of the phases. When they are compounded as a powder, on the other hand, it is difficult to take it out with a puff or the like when the powder is put to use.

In order to decrease the amount of a UV ray absorber percutaneously absorbed and to stably maintain it on the skin, formulating the UV ray absorber in a powdery form is desirable when the make-up cosmetic contains a large amount of powders.

Because of this situation, the development of a long wavelength UV ray absorber has been desired, which is stable, can be formulated with ease in a powdery form, and has a high UV ray masking effect.

The inventors have undertaken extensive studies in order to achieve the above-mentioned objects and as a results have found that a long wavelength UV ray absorber which is stable, can be formulated with ease in a powdery form, and has a high UV ray absorption effect can be obtained by a combined use of the dibenzoylmethane derivative of the above formula (I) and a polyvalent metal oxide.

## SUMMARY OF THE INVENTION

Accordingly, an object of this invention is to provide a long wavelength UV ray absorber comprising: a dibenzoylmethane derivative represented by the general formula:

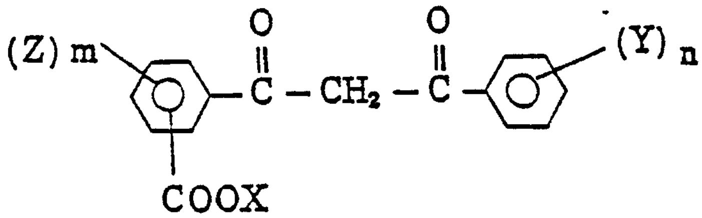

in which X represents a hydrogen atom, a metal cation, an organic cation, a linear or branched aliphatic hydrocarbon group of a $C_{1-24}$ carbon atom content, or a polyoxyalkylene (the alkylene group having 2 or 3 carbon atoms) oxide group of a $C_{1-24}$ carbon atom content, Y and Z may be the same or different and represent a hydroxyl group, a linear or branched aliphatic hydrocarbon group of a $C_{1-24}$ carbon atom content, an alkoxy group of a $C_{1-24}$ carbon atom content, or a polyoxyalkylene (the alkylene group having 2 or 3 carbon atoms) oxide group, and m and n are independently an integer of 0 - 3; and
a polyvalent metal oxide.

Another object of this invention is to provide a cosmetic comprising a long wavelength UV ray absorber which comprises the dibenzoylmethane derivative of the above formula (I) and a polyvalent metal oxide.

Still other object of the present invention is to provide a process for preparing a long wavelength UV ray absorber comprising mixing the dibenzoylmethane derivative of the above formula (I) and a polyvalent metal oxide in water, a lower alcohol, or a mixture thereof.

Other objects, features and advantages of the invention will hereinafter become more readily apparent from the following description.

## DETAILED DESCRIPTION OF THE INVENTION AND PREFERRED EMBODIMENTS

In the dibenzoylmethane derivative of formula (I), as cations represented by X, sodium, potassium, lithium, and the like are named as monovalent metal cations, and arginine, triethanolamine, and the like are mentioned as organic cations.

The dibenzoylmethane derivative represented by formula (I) can be prepared according to known processes. Examples of these processes are the processes described in Ann. Chim. (Rone), 48, 762 (1958), J. Chem. Soc., 2063 (1952), and Pestic Sci., 4, 473, (1973), or the process exemplified in USP No. 4381360.

The process employing the following reaction is the simplest route to prepare this compound:

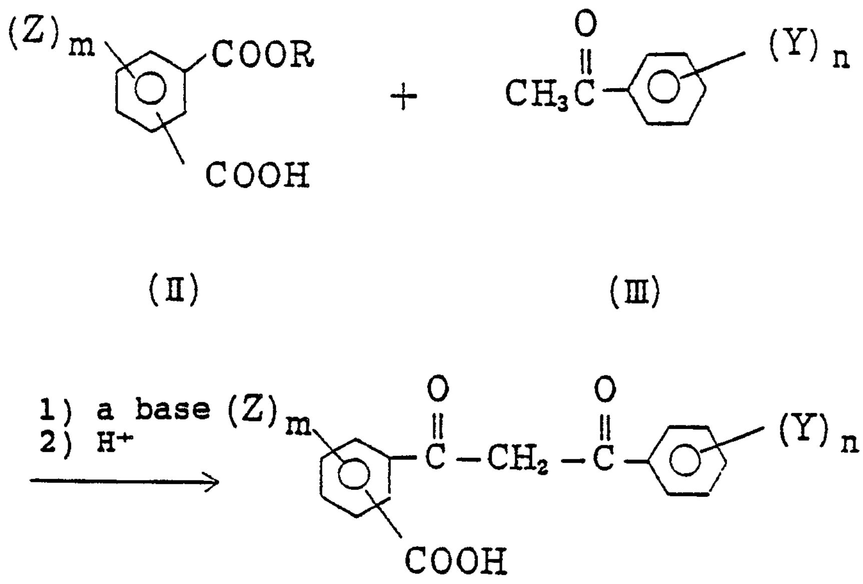


(II) (III)

(Ia)

in which R represents a methyl or ethyl group, Y, Z, m, and n have the same meanings as previously defined in formula (I). In this reaction the monoester of substituted phthalic acid of either ortho, meta, or para isomer (II) and a substituted acetophenone (III) are condensed in the presence of a basic catalyst to produce the compound (Ia). If desired, a salt or ester can be prepared from this compound (a) according to a conventional method.

When the phthalic acid is the ortho isomer, instead of the monoester of the above formula (II) an acid anhydride represented by the following formula (IV) may be used:

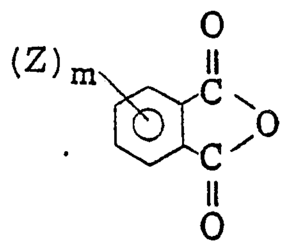

(IV)

in which Z and m have the same meanings as defined above.

The dibenzoylmethane derivative of formula (I) can be grouped into the following compounds (1) - (16).

(1) a dibenzoylmethane carboxylic acid or the salt thereof represented by the following formula (V):

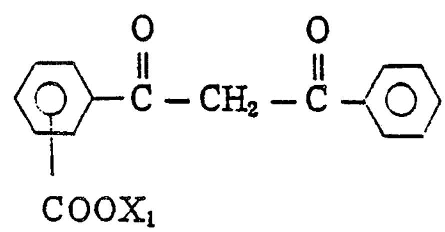

(V)

wherein $X_1$ represents a hydrogen atom, a monovalent metal cation, or an organic cation, and the group $-COOX_1$ may be at either the ortho, meta, or para position.

(2) a 4′-alkoxydibenzoylmethane carboxylic acid or the salt thereof represented by the following formula (VI):

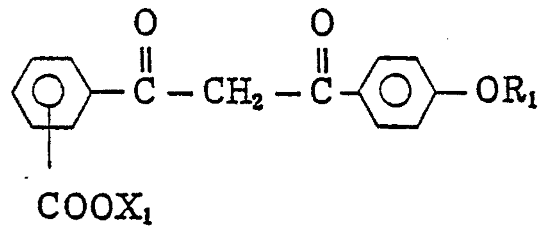

(VI)

wherein $R_1$ represents a hydrogen atom or a hydrocarbon group of a $C_{1-18}$ carbon atom content, preferably a linear or branched, alkyl or alkylene group of a $C_{1-4}$ carbon atom content, and $X_1$ and the position of the group -$COOX_1$ are the same as defined in formula (V).

(3) a 3′-hydroxy-4-alkoxydibenzoylmethane carboxylic acid or the salt thereof represented by the following formula (VII):

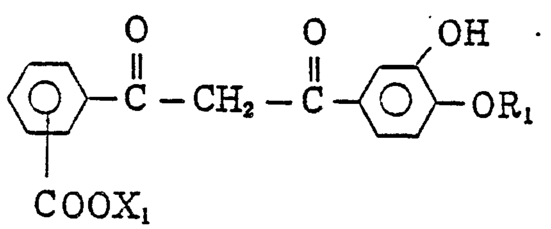

(VII)

wherein $X_1$, $R_1$, and the position of the group -$COOX_1$ are the same as previously defined.

(4) a 2,3′-dihydroxy-4′-alkoxydibenzoylmethane carboxylic acid or the salt thereof represented by the following formula (VIII):

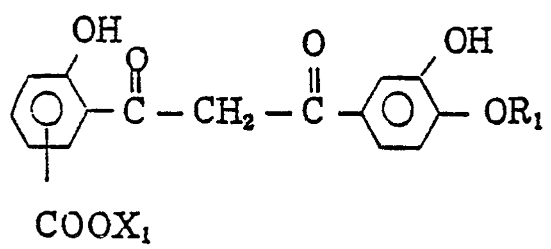

(VIII)

wherein $X_1$, $R_1$, and the position of the group -$COOX_1$ are the same as previously defined.

(5) a 4′-alkoxy-2-polyoxyethyleneoxydibenzoylmethane carboxylic acid or the salt thereof represented by the following formula (IX):

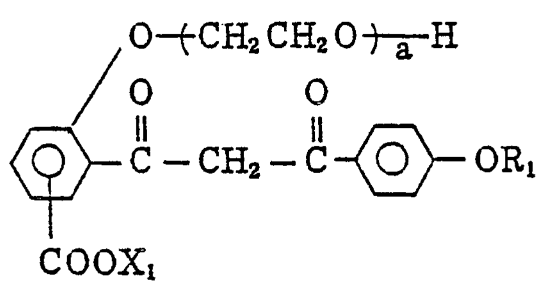

(IX)

wherein $X_1$, $R_1$, and the position of the group -$COOX_1$ are the same as previously defined, and a is an integer of 1 - 10, and preferably of 1 - 6.

(6) a 3′4′-bis(polyoxyethyleneoxy)-dibenzoylmethane carboxylic acid or the salt thereof represented by the following formula (X):

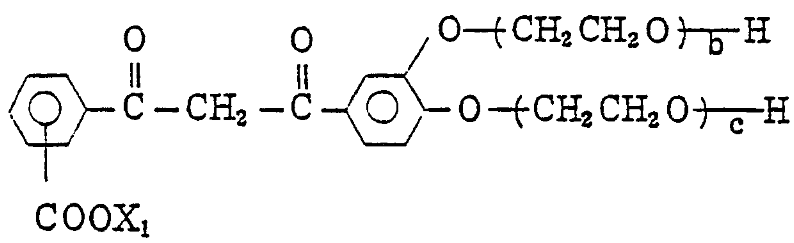

(X)

wherein $X_1$, and the position of the group $-COOX_1$ are the same as previously defined, and b and c independently represent an integer of 1 - 6, and preferably of 1 - 3.

(7) a 4′-polyoxyethyleneoxydibenzoylmethane carboxylic acid or the salt thereof represented by the following formula (XI):

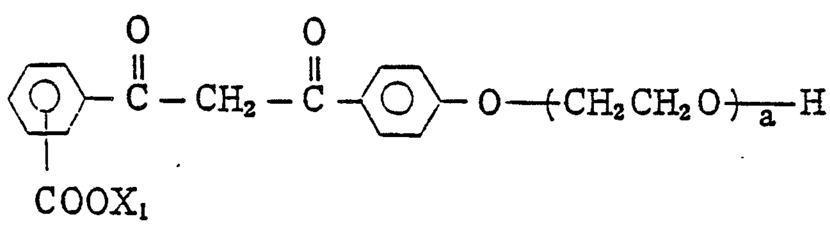

(XI)

wherein $X_1$, a, and the position of the group $-COOX_1$ are the same as previously defined.

(8) a 4′-alkoxy-3′-polyoxyethyleneoxydibenzoylmethane carboxylic acid or the salt thereof represented by the following formula (XII):

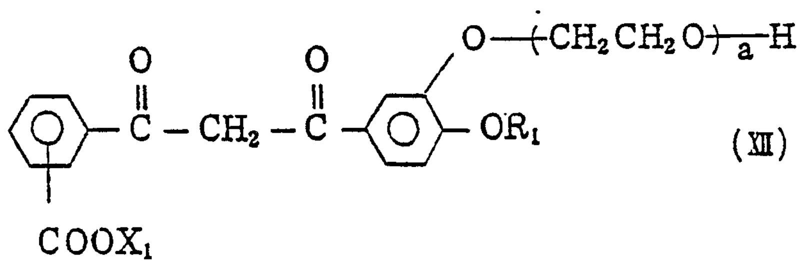

(XII)

wherein $X_1$, $R_1$, a, and the position of the group $-COOX_1$ are the same as previously defined.

(9) a 4′-alkoxy-4-alkyldibenzoylmethane carboxylic acid alkyl ester represented by the following formula (XIII):

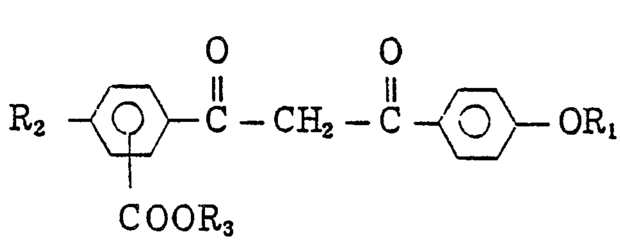

(XIII)

wherein $R_2$ and $R_3$ independently represent a linear or branched, alkyl or alkylene group of a $C_{1-24}$, preferably $C_{1-18}$, carbon atom content, the group $-COOR_3$ is either at ortho or meta position, and $R_1$ has the same meaning as previously defined.

(10) a 3′,4′-dialkoxydibenzoylmethane carboxylic acid alkyl ester represented by the following formula (XIV):

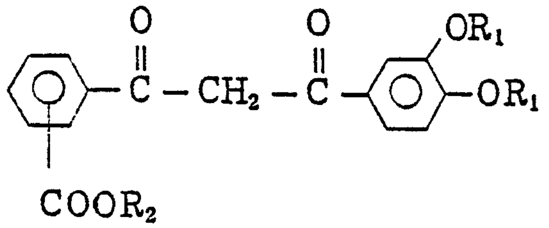

(XIV)

wherein the position of $-COOR_2$ may be either ortho, meta, or para, and $R_1$ and $R_2$ are the same as previously defined.

(11) a 3′,4′-bis(polyoxypropyleneoxy)dibenzoylmethane carboxylic acid alkyl ester represented by the following formula (XV):

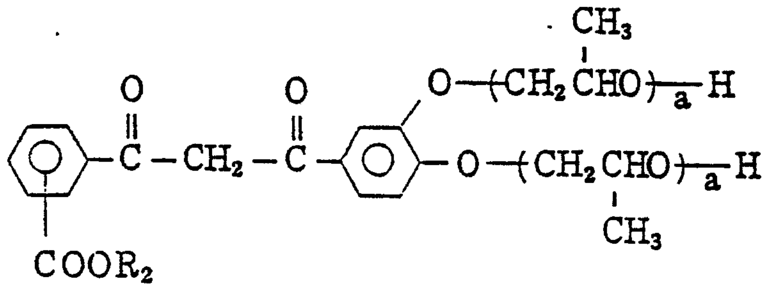

(XV)

wherein a and the position of $-COOR_2$ are the same as previously defined.

(12) a 2,3′-dialkoxy-4′-polyoxypropyleneoxydibenzoylmethane carboxylic acid alkyl ester represented by the following formula (XVI):

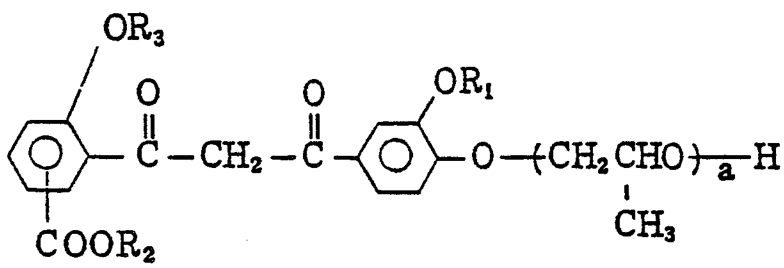

(XVI)

wherein $R_1$, $R_2$, $R_3$, and a, and the position of $-COOR_2$ are the same as previously defined.

(13) a 2,3′,4′-trialkoxydibenzoylmethane carboxylic acid alkyl ester represented by the following formula (XVII):

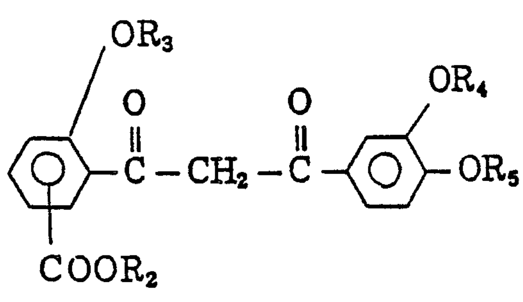

(XVII)

wherein $R_4$ and $R_5$ independently represents a linear or branched, alkyl or alkylene group of a $C_{1-24}$, preferably $C_{1-18}$, carbon atom content, $R_2$ and $R_3$, and the position of $-COOR_2$ are the same as previously defined.

(14) a 4′-alkoxydibenzoylmethane carboxylic acid alkyl ester represented by the following formula (XVIII):

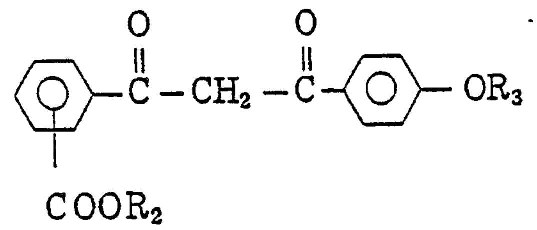

(XVIII)

wherein $R_2$ and $R_3$, and the position of $-COOR_2$ are the same as previously defined.

(15) a 2′,4′-dialkoxydibenzoylmethane carboxylic acid alkyl ester represented by the following formula (XIX):

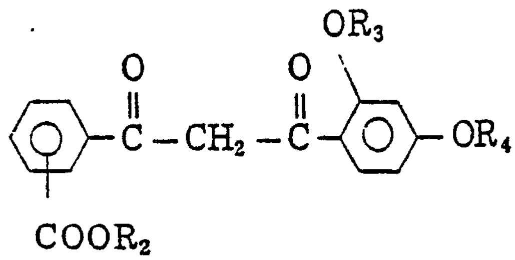

(XIX)

wherein $R_2$, $R_3$, and $R_4$, and the position of $-COOR_2$ are the same as previously defined.

(16) a 4′-alkyldibenzoylmethane carboxylic acid alkyl ester represented by the following formula (XX):

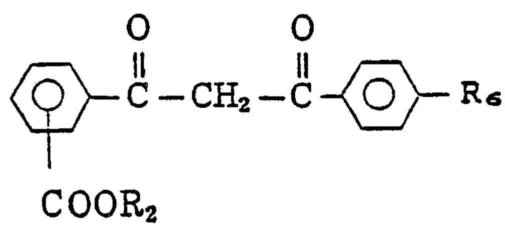

(XX)

wherein $R_6$ represents a hydrogen atom or a linear or branched, aliphatic hydrocarbon group of a $C_{1-24}$ carbon atom content, and $R_2$ and the position of $-COOR_2$ are the same as previously defined.

Among these dibenzoylmethane derivatives, those water soluble ones are, for example, dibenzoylmethane-2-carboxylic acid triethanolamine salt, sodium dibenzoylmethane-3-carboxylate, sodium 4′-methoxydibenzoylmethane-2-carboxylate, sodium 4′-methoxydibenzoylmethane-3-carboxylate, 3′-hydroxy-4′-methoxydibenzoylmethane-2-carboxylic aicd, sodium 2,3′-dihydroxy-4′-methoxydibenzoylmethane-5-carboxylate, potassium 4′-methoxy-2-polyoxyethyleneoxydibenzoylmethane-5-carboxylate, sodium 3′,4′-bis(polyoxyethyleneoxy)-dibenzoylmethane-3-carboxylate, potassium 4′-methoxydibenzoylmethane-4-carboxylate, sodium 4′-polyoxyethyleneoxydibenzoylmethane-4-carboxylate, potassium 4′-methoxy-3′-polyoxyethyleneoxydibenzoylmethane-4-carboxylate, and the like. Enumerated as examples of those oil or fat soluble ones are 4′-methoxy-4-methyldibenzoylmethane-2-carboxylic acid isostearyl ester, 3′,4′-dimethoxydibenzoylmethane-2-carboxylic acid oleyl ester, 3′,4′-bis(polyoxypropyoxy)-dibenzoylmethane-2-carboxylic acid methyl ester, 2,3′-dimethoxy-4′-polyoxypropyleneoxydibenzoylmethane-5-carboxylic acid hexyl ester, 3′,4′-dihexyloxy-2-methoxydibenzoylmethane-5-carboxylic acid butyl ester, 4′-tetracosyloxydibenzoylmethane-3-carboxylic acid methyl ester, 2′,4′-dimethoxydibenzoylmethane-4-carboxylic acid stearyl ester, 4′-tert-butyldibenzoylmethane-4-carboxylic acid-2-ethylhexyl ester, 4′-palmytyldibenzoylmethane-4-carboxylic acid isopropyl ester, and the like.

Given as examples of polyvalent metal oxides are oxides of metal with 2 - 6 valences such as

beryllium, magnesium, calcium, strontium, barium, titanium, vanadium, chromium, manganese, iron, cobalt, nickel, copper, zinc, aluminum, gallium, indium, thallium, zirconium, and the like. Among these, preferable metal oxides to be formulated into cosmetics are oxides of magnesium, calcium, zinc, and zirconium.

It is desirable that these polyvalent metal oxides be fine particles with particle sizes of less than 100 μm, especially of less than 10 μm. Furthermore, it is preferable that these polyvalent metal oxides be present as fine particles without dissolving in the final product, and this should be ensured by the proper selection of the final product substrate.

A typical process for preparing the long wavelength UV ray absorber of this invention is now illustrated. The dibenzoylmethane derivative of formula (I) is first dissolved into a solvent, including water, an alcohol such as methanol, ethanol, mixture of thses alcohols, or the mixture of water and alcohol(s). To this solution, a polyvalent metal oxide, either as is or dispersed into the above solvent, is slowly added, and the mixture is stirred. This operation is carried out at a temperature between room temperature and the boiling point of the solvent. The long wavelength UV ray absorber of this invention can be obtained by removing the solvent from this mixture, followed by drying by a conventional method. The preferable proportion of the dibenzoylmethane derivative of formula (I) and the polyvalent metal oxide is in the range of 0.01 - 100 parts by weight, 0.1 to 10 parts by weight in particular, of the metal oxide per 1 part by weight of the dibenzoylmethane derivative.

The long wavelength UV ray absorber thus prepared has an excellent UV-A absorption capability, and can be applied to various uses requiring UV ray absorption. Especially it can exhibit an outstanding UV ray masking effect to the skin thus protecting the skin from harmful actions of UV ray, when formulated into cosmetics or medicines.

Cosmetics into which the long wavelength UV ray absorber of this invention can be suitably formulated include make-up cosmetics such as foundations, face powders, rouges, and the like, basic cosmetics such as creams, milky lotions, and the like.

Although the amounts of the long wavelength UV ray absorber of this invention to be formulated into these cosmetics are varied depending on the types of the cosmetics, generally 0.1 - 20% by weight, preferably 0.5 -10% by weight, of the dibenzoylmethane derivative of formula (I) is formulated into cosmetics.

The cosmetic of this invention can be prepared by compounding the above long wavelength UV ray absorber of this invention together with known cosmetic bases by means of conventional method. When the cosmetic contains a powder, preferable method of compounding the powder and long wavelength UV ray absorber together is to pulverize and mix them by means of hammer mill or the like. The powders having disk- or plate-type shapes are preferably used in this invention. The long wavelength UV ray absorber can be deposited in an oriented manner onto these disk- or plate-shaped powders by simply pulverizing and mixing such powders and the long wavelength UV ray absorber by means of hammer mill or the like. The cosmetic into which the composite of the disk- or plate-shape powder and the long wavelength UV ray absorber thus prepared is formulated, when applied onto the skin, ia placed onto the skin in an oriented manner with the broader surface of the disk- or plate-shaped powder being in parallel with the surface of the skin. This ensures the effective masking action of the long wavelength UV ray absorber and a higher degree of its UV ray absorption effect. Examples of these disk- or plate-shaped powders are clay minerals such as mica, cerisite, talc, and the like, disk-type silica, disk-type alumina, disk-type polymer, and the like.

Conventionally used cosmetic substrate can be formulated into the cosmetic of this invention. Examples of these cosmetic substrate are body pigments such as kaolin, nylon powder, and the like, inorganic pigments such as titanium oxide, zinc oxide, iron oxide, pearl, and the like, and organic pigment such as Red 202, Red 226, Yellow 4 aluminum lake, and the like. In addition, powders surface-treated with conventional water-repellent agents such as silicone, metal soap, N-acyl glutamic acid, and the like can be employed. Enumerated as examples of oily cosmetic base materials are hydrocarbons such as liquid paraffin, crystal oil, ceresin, ozokerite, montan wax, and the like, oils, fats, or waxes of plant or animal origin such as olive oil, earth wax, carnauba wax, lanolin, spermaceti, and the like, fatty acids and esters thereof such as stearic acid, palmitic acid, oleic acid, glycerin monostearate, glycerin distearate, glycerin monooleate, isopropyl myristate, isopropyl stearate, butyl stearate, and the like, alcohols such as ethyl alcohol, isopropyl alcohol, cetyl alcohol, stearyl alcohol, palmityl alcohol, hexyldodecyl alcohol, and the like. In addition, polyhydric alcohols having humectant effect such as glycol, glycerin, sorbitol, and the like may also be used. Furthermore, in addition to the long wavelength UV ray absorber of this invention, a conventional UV-B absorber may be formulated into the cosmetic composition. The examples of such UV-B absorbers include, p-methylbenzylidene-D, L-campher or its sodium sulfate salt, sodium 2-phenylbenzimidazole-5-sulfonate, sodium 3,4-dimethylphenylglyoxylate, 4-phenylbenzophenone, isooctyl 4-phenylbenzophenone-2′-carboxylate, p-methoxy cinnamate, 2-phenyl-5-methylbenzoxazol, p-dimethyl

aminobenzoate, and the like. Other components conventionally employed in cosmetics, including viscosity increasing agents, antiseptics, antioxidants, and the like may also be formulated into the cosmetic composition of this invention.

The long wavelength UV ray absorber of this invention can effectively shut out UV-A which is harmful to the skin, thus protecting it from the UV-A. It is stable against light and heat over a long period of time. Since the material is sparingly soluble into water, organic solvents, oily substances, or sabum, it is hardly absorbed through the skin. Its effect lasts long as it is hardly removed from the skin by water, sweat, or the like. In addition its capability to disperse into powders such as body pigments with ease through pulverization and mixing, and to attach in an orderly manner onto the powder surfaces ensures its outstanding effect when formulated into a cosmetic composition.

Other features of the invention will become apparent in the course of the following description of the exemplary embodiments which are given for illustration of the invention and are not intended to be limiting thereof.

## EXAMPLES

### Example 1

Preparation of the long wavelength UV ray absorber using 4′-methoxydibenzoylmethane-2-carboxylic acid and magnesium oxide (MgO):

Five (5) g of 4′-methoxydibenzoylmethane-2-carboxylic acid was dissolved into 200 g of ethanol. To this solution was slowly added a suspension of 5 g of magnesium oxide in 50 g of ethanol. The mixture thus obtained was refluxed under heating, followed by evaporation of ethanol therefrom to produce 9.8 g of the above long wavelength UV ray absorber composition in a powdery form.

### Example 2

Preparation of the long wavelength UV ray absorber using 4′-methoxydibenzoylmethane-2-carboxylic acid and calcium oxide (CaO):

The above long wavelength UV ray absorber composition in a powdery form in the amount of 9.5 g was prepared in the same manner as described in Example 1, except that 5 g of calcium oxide was used instead of magnesium oxide.

### Example 3

Preparation of the long wavelength UV ray absorber using 4′-methoxydibenzoylmethane-2-carboxylic acid and zinc oxide (ZnO):

The above long wavelength UV ray absorber composition in a powdery form in the amount of 9.7 g was prepared in the same manner as described in Example 1, except that 5 g of zinc oxide was used instead of magnesium oxide.

### Comparative Example 1

Preparation of a long wavelength UV ray absorber using sodium 4′-methoxydibenzoylmethane-2-carboxylate and α-alumina hydrate:

A suspension of α-alumina hydrate was first prepared by dissolving 5 g of α-alumina hydrate, which was synthesized by hydrolyzing aluminum triisopropoxide, into 150 g of water, the pH of which was adjusted with hydrochloric acid in advance. A solution of 5 g of sodium 4′-methoxydibenzoylmethane-2-carboxylate in 50 g of water was added to the suspension, and the mixture was thoroughly mixed, dehydrated, and dried to produce 9.9 g of the title long wavelength UV ray absorber composition in a powdery form.

Comparative Example 2

Synthesis of magnesium 4′-methoxydibenzoylmethane-2-carboxylate:

Into 100 g of water 6 g of 4′-methoxydibenzoylmethane-2-carboxylic acid and sodium hydroxide (95%) ware dissolved. The pH of the solution obtained was 8.0. To this solution was slowly added an aqueous solution of 3.4 g of magnesium chloride in 50 g of water. The addition of the first solution to the latter was accompanied by addition of a small amount of 1N aqueous solution of sodium hydroxide to maintain the pH of the reaction system above 8. After addition the aqueous solution of magnesium chloride, the precipitate produced was collected by filtration, washed with 0.01N aqueous solution of sodium hydroxide, water, and then methanol to obtain 5.1 g of the title compound as a white powder.

Comparative Example 3

Synthesis of zinc 4′-methoxydibenzoylmethane-2-carboxylate:

The title compound in a yellow powdery form in the amount of 6.04 g was prepared in the same manner as described in Comparative Example 2, except that 7.0 g of zinc acetate dihydrate was used instead of magnesium chloride.

Test Example

The relative diffusion reflective spectrum was measured on 4′-methoxydibenzoylmethane-2-carboxylic acid, and the long wavelength UV ray absorbers prepared in Examples 1 - 3 and Comparative Examples 1 - 3, by diluting each of the powders with barium sulfate and using integrating sphere on an automatic recording spectrophotometer (MPS-2000; manufactured by Shimazu Co., Ltd.). All the samples measured had the minimum reflection in the neighborhood of 350 nm. Kubel-Munk extinction coefficients k [J. Opt. Soc. Am., 38, 448 (1948); 38, 1067 (1948); 44, 330 (1954)] of 4′-methoxydibenzoylmethane-2-carboxylic acid and each of its derivatives in respective samples were determined based on their reflections at the minimum reflection wavelength. The results are shown in Table 1.

TABLE 1

| Samples | The minimum reflection wavelength (nm) | Extinction coefficient (k) |
|---|---|---|
| Long wavelength UV ray absorber of Example 1 | 344 | 4.0 |
| Long wavelength UV ray absorber of Example 2 | 346 | 4.0 |
| Long wavelength UV ray absorber of Example 2 | 347 | 4.4 |
| Long wavelength UV ray absorber of Comparative Example 1 | 347 | 3.5 |
| Long wavelength UV ray absorber of Comparative Example 2 | 345 | 1.1 |
| Long wavelength UV ray absorber of Comparative Example 3 | 346 | 1.1 |
| 4′-methoxydibenzoylmethane-2-carboxylic acid | 347 | 0.8 |

Example 4

Powder Foundation

<Formulation>

| | (%) |
|---|---|
| (1) Mica | Balance |
| (2) Long wavelength UV ray absorber of Example 3 | 10 |
| (3) Talc | 20 |
| (4) Titanium oxide | 10 |
| (5) Red iron oxide | 0.8 |
| (6) Yellow iron oxide | 2.5 |
| (7) Black iron oxide | 0.1 |
| (8) Liquid paraffin | 8 |
| (9) Bees wax | 2 |
| (10) Antiseptic | Appropriate amount |
| (11) Perfume | Small amount |

<Method of Preparation>

Components (1) - (7) were mixed and pulverized, and transferred into a high speed blender, to which a mixture of the components (8) - (10) heated at 80°C for dissolution was slowly added and blended to homogenize. To this mixture the component (11) was added and blended. This blended mixture was again pulverized and passed through a sieve. The sieved substance was press-molded in a metal mold pan.

Example 5

Creamy Foundation

<Formulation>

| | (%) |
|---|---|
| (1) Stearic acid | 5 |
| (2) Lipophilic-type monostearic acid glycerol | 2.5 |
| (3) Setostearyl alcohol | 1 |
| (4) Monolauric acid propylene glycol | 3 |
| (5) Squalan | 7 |
| (6) Olive oil | 8 |
| (7) Purified water | Balance |
| (8) Antiseptic | Appropriate amount |
| (9) Triethanolamine | 1.2 |
| (10) Sorbit | 3 |
| (11) Titanium oxide | 10 |
| (12) Talc | 5 |
| (13) Coloring pigment | Appropriate amount |
| (14) Long wavelength UV ray absorber of Example 1 | 8 |
| (15) Perfume | Small amount |

<Preparation>

Components (11) - (14) were mixed and pulverized. An aqueous solution comprising components (7) - (10) was separately prepared, into which the pigments were added to disperse, and the dispersion was heated at 75°C. The oil components (1) - (6) which were mixed together and heated at 80°C to dissolution was added to this aqueous dispersion, while stirring to emulsify the whole mixture. The emulsion was cooled while stirring to 50°C, at which temperature component (15) was added. The stirring was further continued to cool the mixture.

Example 6

Oil foundation:

<Formulation>

| | (%) |
|---|---|
| (1) Long wavelength UV ray absorber of Example 2 | 10 |
| (2) Talc | Balance |
| (3) Kaolin | 12 |
| (4) Titanium oxide | 13 |
| (5) Red iron oxide | 1.5 |
| (6) Yellow iron oxide | 2.0 |
| (7) Black iron oxide | 0.5 |
| (8) Liquid paraffin | 15 |
| (9) Isopropyl palmitate | 10 |
| (10) Lanolin alcohol | 3 |
| (11) Microcrystalline wax | 7 |
| (12) Ozokerite | 8 |
| (13) Antiseptic | Appropriate amount |
| (14) Perfume | Appropriate |

<Preparation>

Components (1) - (7) were mixed and pulverized, and were slowly added and thoroughly dispersed into oil phase components (9) - (13), which were mixed and heated at 80 °C in advance. After addition of component (14) to this mixture and blending, the whole mixture was filled into a metal mold and cooled.

Example 7

O/W type Cream:

<Formulation>

| | (%) |
|---|---|
| (1) Bees wax | 5.5 |
| (2) Cetanol | 4.5 |
| (3) Hydrogenated lanolin | 7 |
| (4) Squalan | 33 |
| (5) Fatty acid glycerol | 3.5 |
| (6) Lipophilic-type monostearic acid glycerol | 2 |
| (7) Polyoxyethylene sorbitane monolauric acid ester (20 E.O.) | 2 |
| (8). Long wavelength UV ray absorber of Example 3 | 8 |
| (9) Perfume | Small amount |
| (10) Antiseptic | Appropriate amount |
| (11) Antioxidant | Appropriate amount |
| (12) Propylene glycol | 4.5 |
| (13) Purified water | Appropriate amount |

<Preparation>

Water phase components (8), (10), (12), and (13) were mixed while stirring and were maintained at 80 °C. Other components were mixed altogether and heated to dissolution to produce an oil phase, which was maintained at 80 °C. The above water phase components were added to this oil phase to effect preliminary emulsification. This emulsion was then thoroughly emulsified with homogenizer and cooled to 30 °C to produce the product.

Obviously, numerous modifications and variations of the present invention are possible in light of the above teachings. It is therefore to be understood that the scope of the appended claims, the invention may be practiced otherwise than as specifically described herein.

## Claims

1. A long wavelength UV ray absorber comprising:
a dibenzoylmethane derivative represented by the general formula:

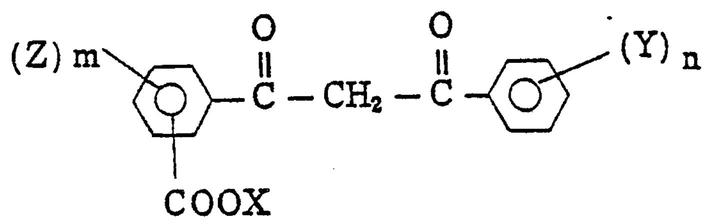

in which X represents a hydrogen atom, a metal cation, an organic cation, a linear or branched aliphatic hydrocarbon group of a $C_{1-24}$ carbon atom content, or a polyoxyalkylene (the alkylene group having 2 or 3 carbon atoms) oxide group of a $C_{1-24}$ carbon atom content, Y and Z may be the same or different and represent a hydroxyl group, a linear or branched aliphatic hydrocarbon group of a $C_{1-24}$ carbon atom content, an alkoxy group of a $C_{1-24}$ carbon atom content, or a polyoxyalkylene (the alkylene group having 2 or 3 carbon atoms) oxide group, and m and n are independently an integer of 0 - 3; and
a polyvalent metal oxide.

2. A cosmetic comprising a long wavelength UV ray absorber which comprises:
a dibenzoylmethane derivative represented by the general formula:

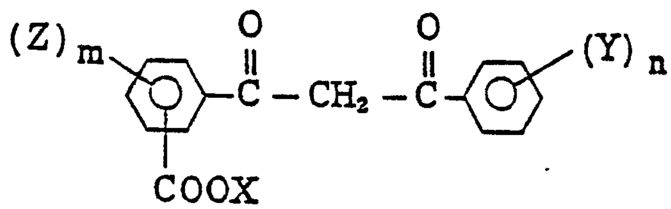

in which X represents a hydrogen atom, a metallic cation, an organic cation, a linear or branched aliphatic hydrocarbon group of a $C_{1-24}$ carbon atom content, a polyoxyalkylene (the alkylene group having 2 or 3 carbon atoms) oxide group of a $C_{1-24}$ carbon atom content, Y and Z may be the same or different and represent a hydroxyl group, a linear or branched aliphatic hydrocarbon group of a $C_{1-24}$ carbon atom content, an alkoxy group of a $C_{1-24}$ carbon atom content, or a polyoxyalkylene (the alkylene group having 2 or 3 carbon atoms) oxide group, and m and n are independently an integer of 0 - 3; and
a polyvalent metal oxide.

3. A process for preparing a long wavelength UV ray absorber comprising: mixing
(a) a dibenzoylmethane derivative represented by the general formula:

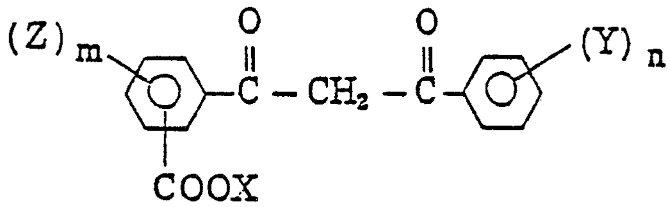

in which X represents a hydrogen atom, a metallic cation, an organic cation, a linear or branched aliphatic hydrocarbon group of a $C_{1-24}$ carbon atom content, a polyoxyalkylene (the alkylene group having 2 or 3 carbon atoms) oxide group of a $C_{1-24}$ carbon atom content, Y and Z may be the same or different and represent a hydroxyl group, a linear or branched aliphatic hydrocarbon group of a $C_{1-24}$ carbon atom content, an alkoxy group of a $C_{1-24}$ carbon atom content, or a polyoxyalkylene (the alkylene group having 2 or 3 carbon atoms) oxide group, and m and n are independently an integer of 0 - 3, and

(b) a polyvalent metal oxide, in water, a lower alcohol, or a mixture thereof.

European Patent Office

# EUROPEAN SEARCH REPORT

Application Number

EP 88 11 3171

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | EP-A-0 154 928 (KAO CORP.)<br>* Whole document * | 1-3 | A 61 K 7/42 |
| A | EP-A-0 193 387 (PROCTER & GAMBLE)<br>* Pages 7-8; examples * | 1-3 | |
| A | DE-A-2 533 497 (HENSELWERK)<br>* Claims * | 1-3 | |
| X,D | PATENT ABSTRACTS OF JAPAN, vol. 11, no. 53 (C-404)[2500], 19th February 1987; & JP-A-61 213 280 (KAO CORP.) 22-09-1986<br>* Abstract * | 1-3 | |
| | | | TECHNICAL FIELDS SEARCHED (Int. Cl.4) |
| | | | A 61 K |

The present search report has been drawn up for all claims

| Place of search | Date of completion of the search | Examiner |
|---|---|---|
| THE HAGUE | 10-10-1988 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)